(19) Europäisches Patentamt
European Patent Office
Office européen des brevets

(11) Numéro de publication: **0 117 171**
**B1**

(12) # FASCICULE DE BREVET EUROPEEN

(45) Date de publication du fascicule du brevet: **01.07.87**

(51) Int. Cl.$^4$: **C 07 D 237/20,** C 07 D 409/04

(21) Numéro de dépôt: **84400102.4**

(22) Date de dépôt: **18.01.84**

(54) **Dérivés aminés de la pyridazine substitués en position 6 par un hétérocycle ou un alicycle, procédé d'obtention et médicaments les contenant.**

(30) Priorité: **24.01.83 FR 8301029**

(43) Date de publication de la demande:
**29.08.84 Bulletin 84/35**

(45) Mention de la délivrance du brevet:
**01.07.87 Bulletin 87/27**

(84) Etats contractants désignés:
**AT BE CH DE FR GB IT LI LU NL SE**

(56) Documents cités:
**FR-A-2 141 697**

**CHEMICAL ABSTRACTS, vol. 76, no. 7, 14 février 1972, page 22, no. 41974h, Columbus, Ohio, US M.R. ORNELLAS: "Biochemical studies of cerebral subfractions after chronic administration of 4-methyl-3 (2-(morpholino)ethylamino)-6-phenylpyridazine hydrochloride, AG620"**

(73) Titulaire: **SANOFI, société anonyme**
**40, Avenue George V**
**F-75008 Paris (FR)**

(72) Inventeur: **Kan, Jean-Paul**
**25 Lotissement l'Olivette**
**F-34100 Clapiers (FR)**
Inventeur: **Biziere, Kathleen**
**6, Lotissement Rayons d'Oc**
**F-34170 Clapiers (FR)**
Inventeur: **Wermuth, Camille-Georges**
**3, rue de la Côte d'Azur**
**F-67100 Strasbourg (FR)**

(74) Mandataire: **Gillard, Marie-Louise et al**
**Cabinet Beau de Loménie 55, Rue d'Amsterdam**
**F-75008 Paris (FR)**

(58) References cited:
**CHEMICAL ABSTRACTS, vol. 73, no. 1, 6 juillet 1970, page 199, no. 12845z, Columbus, Ohio, US M.R. ORNELLAS et al.: "Pharmacological interpretation of the energy metabolism of rat brain in vivo and in vitro in connection with a study on some pyridazines"**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 117 171 B1

Courier Press, Leamington Spa, England.

**Description**

La présente invention concerne des dérivés de la pyridazine; elle concerne également un procédé de préparation desdits dérivés et les médicaments qui contiennent comme principe actif au moins un desdits dérivés.

Des dérivés de la pyridazine substitués en position 6 par un reste aromatique ont été décrits dans la demande FR 2 141 697.

Les composés selon l'invention sont substitués en position 6 par un hétérocycle ou un alicycle; cette modification structurale entraîne une modification du spectre d'activité. Les composés selon l'invention présentent une forte composante sérotoninergique.

Ils répondent à la formule générale:

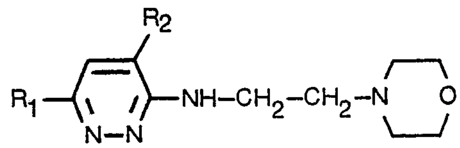

(I)

dans laquelle:

$R_1$ désigne un groupe thiényle-2, thiényle-3 ou cyclohexyle;

$R_2$ représente un groupe alkyle comportant de 1 à 4 atomes de carbone ou un atome d'hydrogène ainsi que les sels desdits dérivés avec les acides.

La présente invention concerne également les sels d'addition que fournissent les composés (I) avec les acides minéraux ou organiques pharmaceutiquement acceptables.

Elle comprend également un procédé de préparation des composés de formule (I) et des sels d'acides correspondants ainsi que leur application en thérapeutique.

Les composés selon l'invention sont obtenus à partir d'une chloro-3 pyridazine convenablement substituée *1* par action de l'amine

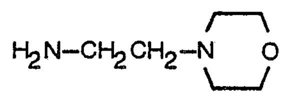

2

selon le schéma réactionnel:

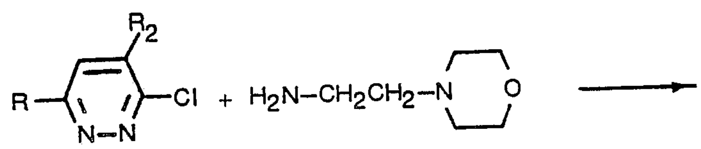

(I)

1 2

La réaction entre le dérivé chloré *1* et l'amine *2* s'effectue par chauffage au sein d'un solvant convenable, tel qu'un alcool, le plus souvent à température d'ébullition du solvant. On effectue la réaction en présence d'un accepteur d'hydracide destiné à fixer l'acide chlorhydrique formé; le plus souvent, on utilise comme tel un excès de l'amine *2*.

On isole le produit (I) par dilution du mélange réactionnel avec de l'eau et extraction par un solvant convenable, tel que l'acétate d'éthyle ou l'éther.

Les composés (I) peuvent être salifiés de façon habituelle par action de l'acide sur une solution chaude de la base, le solvant étant choisi de façon que le sel cristallise par refroidissement ou par addition d'un autre solvant.

Les dérivés chlorés *1* peuvent être préparés selon des méthodes connues notamment selon le schéma réactionnel suivant:

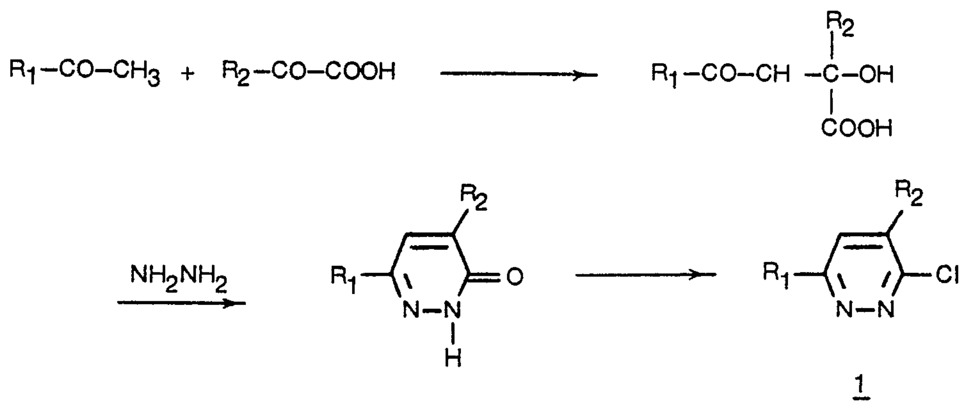


1

qui consiste à condenser la cétone $R_1$—CO—$CH_3$ avec l'α-cétoacide $R_2$—CO—COOH pour obtenir l'acide α-hydroxygammacétonique correspondant. Par action de l'hydrazine, ce dernier conduit à la pyridazone-3 corpondante qui à son tour, par action de l'oxychlorure de phosphore, fournit la chloro-3 pyridazine *1*.

## Exemple 1

Morpholinoéthylamino-3 méthyl-4 (thiényl-2)-6 pyridazine, dichlorhydrate; (CM 30387)

$R_1$ = 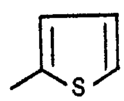 ; $R_2$ = $CH_3$ (I)

a) Acide hydroxy-2 méthyl-2 (thénoyl-2)-3 propanoïque

On neutralise 70,4 g d'acide pyruvique par une solution de potasse à 20% en agitant et en refroidissant, puis on ajoute 101 g d'acétyl-2 thiophène. On ajoute ensuite une solution de 56 g de potasse dans 1600 ml de méthanol sous agitation puis on abandonne le mélange au réfrigérateur pendant 4 jours.

On acidifie la solution par l'acide sulfurique 10 N jusqu'à pH 3—4. On filtre le précipité de sulfate de potassium et on évapore le méthanol au bain-marie sous vide. On acidifie la solution restante par l'acide sulfurique 10 N et on ajoute de l'eau pour dissoudre le sulfate de potassium. On extrait avec de l'éther et on lave la phase éthérée deux fois avec 100 ml d'eau. On extrait la solution organique avec une solution aqueuse de bicarbonate de potassium à 10%. On sépare la phase aqueuse qu'on extrait avec un peu d'éther, puis on l'acidifie par addition d'acide sulfurique 10 N.

L'acide attendu cristallise; on l'essore et on le lave avec un peu d'éther isopropylique. On l'utilise tel quel pour l'étape suivante.

b) Méthyl-4 (thiényl-2)-6 pyridazone-3

On dissout 20 g de l'acide obtenu ci-dessus dans 200 ml de butanol puis on ajoute 7 g d'hydrate d'hydrazine.

On porte le mélange au reflux en distillant lentement l'azéotrope butanol-eau formé. Lorsque la formation d'eau a cessé, on distille environ 160 ml de butanol et on laisse cristalliser par refroidissement.

On essore les cristaux et on recristallise dans l'acide acétique. Poids 14,5 g; Point de fusion 236°C.

c) Chloro-3 méthyl-4 (thiényl-2)-6 pyridazine

On chauffe au bain-marie pendant 3 heures le mélange de 14 g de pyridazone obtenue ci-dessus et 90 ml d'oxychlorure de phosphore. On verse le mélange sur de la glace pilée et on alcalinise par une solution de soude à 20%.

On essore le solide et on recristallise dans le méthanol. Poids 10,5 g; Point de fusion 146°C.

d) CM 30387

On chauffe au reflux pendant 3 jours le mélange de 10,11 g de chloropyridazine obtenue ci-dessus et 18,9 g de morpholino-2 éthylamine dans 150 ml de butanol.

On verse la solution dans l'eau et on alcalinise avec une solution de soude. On extrait avec de l'éther, on sèche sur sulfate de sodium et concentre à siccité sous vide.

Le résidu cristallise et on le recristallise dans l'acétate d'éthyle. Poids 9 g; point de fusion: 132°C.

Dichlorhydrate

A la solution de 8,9 g de la base ci-dessus dans 50 ml d'isopropanol, on ajoute 5,5 ml d'une solution aqueuse d'acide chlorhydrique concentré. On essore le solide formé et on le recristallise dans de l'éthanol absolu. Poids 10,6 g; point de fusion: 234°C.

Exemple 2

Morpholinoéthylamino-3 méthyl-4 (thiényl-3)-6 pyridazine, dichlorhydrate; (CM30388)

$R_1$ = 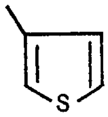 ; $R_2 = CH_3$ (I)

En opérant comme dans l'exemple 1, mais en remplaçant l'acétyl-2 thiophène par l'acétyl-3 thiophène, on obtient successivement:

l'acide hydroxy-2 méthyl-2 (thénoyl-3)-3 propanoïque;

la méthyl-4 (thiényl-3)-6 pyridazone-3; point de fusion: 246°C (acide acétique);

la chloro-3 méthyl-4 (thiényl-3)-6 pyridazine; point de fusion: 171°C (dioxanne);

le CM 30388;

Base: F. 56°C (acétate d'éthyle — éther de pétrole) Dichlorhydrate: point de fusion: 152°C (alcool absolu).

Exemple 3

Morpholinoéthylamino-3 méthyl-4 cyclohexyl-6 pyridazine, dichlorhydrate; (CM 30390)

$R_1$ = 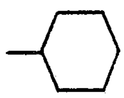 ; $R_2 = CH_3$ (I)

En opérant comme dans l'exemple 1, mais en remplaçant l'acétyl-2 thiophène par la cyclohexylméthylcétone, on obtient successivement:

l'acide hydroxy-2 méthyl-2 cyclohexyl-3 propanoïque;

la méthyl-4 cyclohexyl-6 pyridazone-3; point de fusion: 173°C (isopropanol-éther isopropylique);

la chloro-3 méthyl-4 cyclohexyl-6 pyridazine chromatographié sur silice en éluant avec acétate d'éthylehexane (25—75 vol/vol);

CM 30390;

Base: huile jaune

Dichlorhydrate: point de fusion: 237°C (isopropanol-éther).

Les produits selon l'invention ont été soumis à des essais pharmacologiques en vue de déterminer leur activité sur le système nerveux central. On indiquera ci-après les diverses épreuves auxquelles ont été soumis les produits.

I — Activite Antidepressive

Antagonisme de la ptôse induite par la réserpine

Ce test décrit par GOURET (Journal de Pharmacologie, Paris, 1973, *4* (1), 105—128) a été réalisé chez la souris femelle CDI (Charles River) pesant 20±1 g. La réserpine entraîne un ptôsis 1 heure après son administration intraveineuse; certains antidépresseurs s'opposent à ce ptôsis.

Le protocole suivant a été choisi:

Les substances à étudier ont été administrées i.p. La réserpine est administrée simultanément par voie intraveineuse à la dose de 2 mg/kg. 1 heure après l'administration de réserpine, on note le nombre d'animaux ne présentant pas de ptôsis.

Ce test a été réalisé sur des lots de 10 souris, les résultats sont exprimés en pourcentage d'animaux ne présentant pas de ptôsis et sont la moyenne de, au moins, deux expériences.

Potentialisation des "head twitches" induits par le 5-hydroxytryptophane

Le 5-hydroxytryptophane précurseur de la 5-hydroxytryptamine induit chez la souris un comportement typique caractérisé par de brusques saccades des oreilles (head twitches). Les molécules qui activent la transmission sérotoninergique centrale augmentent le nombre des "head twitches", alors que les antidépresseurs tricycliques sont inactifs.

Au temps zéro, le produit à étudier est administré par voie intrapéritonéale puis, 1 heure après, une dose unique (200 mg/kg) de 1-5-hydrotryptophane en suspension dans l'eau distillée est administrée à son tour par la même voie. Chaque animal (souris femelle CDI Charles River pesant de 22 à 24 g) est immédiatement placé dans un bécher cylindrique en verre. On compte durant 20 minutes le nombre de "head twitches".

Pour chaque dose du produit à tester, on opère sur des lots de 10 animaux en comparaison avec un lot témoin qui ne reçoivent que le véhicule.

Les résultats sont exprimés en % par rapport au score moyen obtenu chez les souris témoins.

Mesure de l'activité MAO A in vitro

L'activité inhibitrice de la monoamine oxydase A est estimée selon la méthode décrite par KAN et al. (Life Sciences *26*, 2165—2171, 1980) en utilisant comme source d'enzyme le striatum de rat.

Après dissection, les tissus sont homogénéisés dans 16 volumes (poids par volume) de tampon phosphate glacé (0,1 M, pH = 7,40). Des fractions de 0,1 ml sont incubées pendant 10 minutes en présence de 5-hydroxytryptamine marquée au carbone 14 (concentration finale 480 µM) et de concentrations variables du produit à tester; le volume final d'incubation est de 0,5 ml. Les métabolites acides sont extraits dans 7 ml d'un mélange toluène-acétate d'éthyl 1—1 (vol/vol) puis comptés par scintillation liquide. On détermine graphiquement la concentration du produit testé qui inhibe de 50% C.I. 50) l'activité témoin.

II — Activite Dopaminergique

Elle a été étudiée par l'analyse du comportement de rotation chez la souris après lésion unilatérale du striatum (P. Protais et al., J. Pharmacol, 1976, *7*, 251).

Des souris femelles Charles River CDI pesant de 20 à 24 g ont préablement fait l'objet d'une lésion unilatérale du striatum par injection stéréotaxique de 6-hydroxydopamine à raison de 8 mcg par animal. Une semaine après cette opération, les produits à étudier sont administrés par voie intrapéritonéale à des groupes de 7 souris. 1 heure après l'administration du produit, le nombre des rotations est déterminé pendant une période de 2 minutes. Les rotations ipsilatérales à la lésion sont comptées positivement et les rotations contralatérales sont comptés négativement.

La somme algébrique des rotations pour un groupe d'animaux traités est comparés à celle du groupe d'animaux témoins n'ayant reçu que le véhicule (sérum physiologique).

Les résultats sont exprimés en variation pour cent des rotations des animaux traités par rapport aux animaux témoins.

III — Activite Cholinergique

Les récepteurs cholinergiques de type muscarinique peuvent être marqués in vitro par le quinuclidinyl-benzylate tritié (QNB[$^3$H]). Ce marquage est réalisé selon la technique décrite par YAMAMURA et collaborateurs (Proceedings of the National Academy of Sciences of the USA, *71*, 1725—9 (1974).

Des cerveaux de rat dont on a retiré le cervelet sont homogénéisés dans 10 volumes (poids par volume) de sucrose 0,32 M, puis centrifugés à 1000 × g pendant 10 minutes. On élimine le culot et on réhomogénéise le surnageant.

Des fractions aliquotes de 0,1 ml sont incubées à 25°C pendant 1 heure dans 2 ml de tampon phosphate (0,05 M, pH = 7,4) contenant des concentrations variables en produit à tester et 0,05 nM de QNB ($^3$H).

Les échantillons sont filtrés ensuite sur filtres Whatman GF/B sous léger vide puis lavés par le tampon d'incubation. La radioactivité absorbée sur les filtres est comptée par scintillation liquide.

La fixation non spécifique est déterminée en présente de 100 µM d'oxotrémorine. On détermine graphiquement les CI 50.

Les résultats obtenus avec les produits de l'invention sont réunis dans le tableau ci-après.

Dans ce tableau, on a fait figurer également les résultats obtenus sur les mêmes tests avec un produit de l'art antérieur appartenant à la famille chimique des pyridazines, désigné sous le nom de Minaprine (DCI) et répondant à la formule:

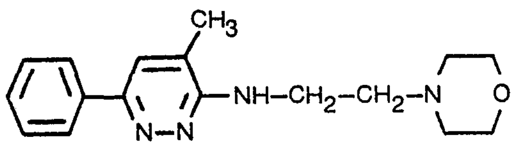

Les résultats obtenus avec les produits de l'invention montrent que:

dans l'axe antidépresseur ils présentent une meilleurs activité sérotoninomimétique (test au 5—HTP) et une meilleure activité inhibitrice de la monoamine oxydase que le produit de référence;

ils sont plus actifs comme agents cholinomimétiques (liaison au $^3$H—QNB) que le produit de référence;

ils sont à l'inverse du produit de référence dépourvus de toute activité dopaminomimétique.

TABLEAU

| Produit No. | Ptôse à la Réserpine ED 50 mg/kg | Test au 5-HTP % variation (dose en mg/kg) | Inhibition MAO A ($IC_{50}$, mg/kg) | Liaison au QNB ($^3H$) ($IC_{50}$, M) | Test des rotations % variation (dose en mg/kg) |
|---|---|---|---|---|---|
| CM 30 387 | 4,8 | 632 ** (8) | 10 | $2{,}4 \times 10^{-5}$ | Inactif à 10 |
| CM 30 388 | 10,3 | 647 ** (8) | 7,8 | $1{,}8 \times 10^{-5}$ | Inactif à 10 |
| CM 30 390 | 4,5 | 219 * (7,5) | — | $1{,}4 \times 10^{-5}$ | Inactif à 10 |
| Minaprine | 5 | 257 * (10) | 16 | $10^{-4}$ | – 79 ** (0,5) |

* p 0,05
** p 0,01

Par ailleurs les produits selon l'invention sont peu toxiques. Ils pourront donc être utilisés en médecine humaine pour le traitement des dépressions et états dépressifs de toute nature.

Ces produits peuvent être administrés par voie orale, par voi rectale ou par voie injectable. Les compositions pharmaceutiques les contenant peuvent être solides ou liquides et se présenter sous forme de comprimés, gélules, granulés, suppositoires ou préparations injectables.

La posologie peut varier dans de larges proportions suivant le mode d'administration et suivant le type et la gravité de l'affection à traiter.

Chez l'adulte, par voie orale, elle est le plus souvent comprise entre 0,010, et 0,500 g, éventuellement répartie en plusieurs prises.

A titre d'exemple, on peut indiquer la préparation galénique suivant:

GELULES

| | |
|---|---|
| CM 30388 | 100 mg |
| Aérosil | 0,5 mg |
| Stéarate de magnésium | 1,5 mg |
| Amidon STA RX 1500 | 48 mg |
| | 150 mg |

**Revendications pour les Etats contractants: BE CH DE FR GB IT LI LU NL SE**

1. Dérivés aminés de la pyridazine de formule:

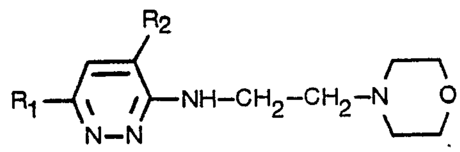

(I)

dans laquelle:

$R_1$ désigne un groupe thiényle-2, thiényle-3 ou cyclohexyle;

$R_2$ représente un groupe alkyl comportant de 1 à 4 atomes de carbone ou un atome d'hydrogène ainsi que les sels desdits dérivés avec les acides.

2. Procédé de préparation des dérivés aminés de la pyridazine selon la revendication 1 caractérisé en ce que l'on fait réagir un dérivé chloré de formule:

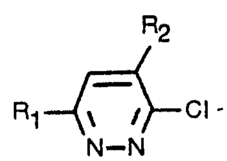

sur l'amine de formule:

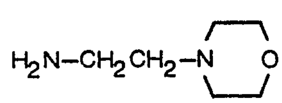

la réaction étant effectuée dans un solvant, à chaud en présence d'un accepteur d'hydracide, et en ce qu'éventuellement on transforme le composé obtenu en l'un de ses sels par action d'un acide.

3. Procédé selon la revendication 2, caractérisé en ce que le solvant employé est un alcool.

4. Médicaments caractérisés en ce qu'ils contiennent au moins un des dérivés aminés de la pyridazine de formule (I) ou un de leurs sels d'acide pharmaceutique acceptables correspondants.

5. Médicaments selon la revendication 4, caractérisés en ce qu'ils sont actifs sur le système nerveux central.

6. Médicaments selon l'une des revendications 4 ou 5, caractérisés en ce qu'ils sont conditionnés en vue d'une administration par voie orale et qu'ils contiennent de 0,01 à 0,50 g de dérivé de formule I ou d'un sel d'acide pharmaceutiquement acceptable correspondant.

**Revendications pour l'Etat contractant: AT**

1. Procéde pour la préparation de dérivés aminés de la pyridazine de formule:

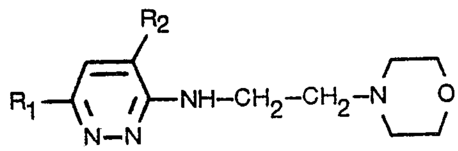

(I)

dans laquelle:
$R_1$ désigne un groupe thiényle-2, thiényle-3 ou cyclohexyle;
$R_2$ représente un groupe alkyle comportant de 1 à 4 atomes de carbone ou un atome d'hydrogène, ainsi que des sels desdits dérivés avec les acides, caractérisé en ce que l'on fait réagir un dérivé chloré de formule:

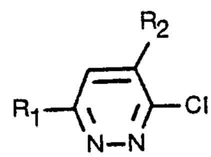

sur l'amine de formule:

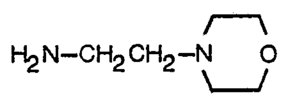

la réaction étant effectuée dans un solvant, à chaud en présence d'un accepteur d'hydracide et en ce qu'éventuellement on transforme le composé obtenu en l'un de ses sels par action d'un acide.

2. Procédé selon la revendication 1, caractérisé en ce que le solvant employé est un alcool.

3. Utilisation des dérivés aminés de la pyridazine de formule (I) et de leurs sels d'acide pharmaceutiquement acceptables correspondants, pour la préparation de médicaments.

4. Utilisation selon la revendication 3, pour la préparation de médicaments actifs sur le système nerveux central.

5. Utilisation selon l'une des revendications 3 ou 4, pour la préparation de médicaments, conditionnés en vue d'une administration par voie orale et contenant de 0,01 à 0,50 g de dérivé de formule (I) ou d'un sel d'acide pharmaceutiquement acceptable correspondant.

**Claims for the Contracting States: BE CH DE FR GB IT LI LU NL SE**

1. Aminated derivatives of pyridazine of formula:

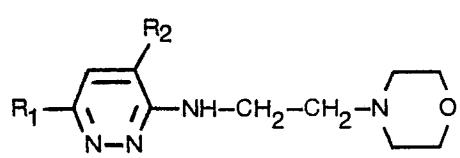

(I)

in which:
$R_1$ designates a 2-thienyl, 3-thienyl or cyclohexyl group;
$R_2$ represents an alkyl group having from 1 to 4 atoms of carbon, or an atom of hydrogen as well as the salts of said derivatives with the acids.

2. Process for obtaining aminated derivatives of pyridazine according to claim 1, characterized in that it comprises the steps of reacting a chlorinated derivative of formula:

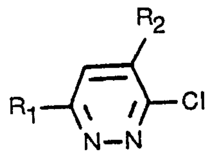

with an amine of formula:

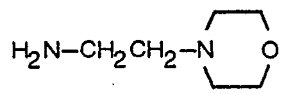

the reaction being carried out in a solvent, hot in the presence of a hydracid acceptor, and possibly of converting the derivative thus obtained into a salt thereof by action of an acid.

3. Process according to claim 2, characterized in that the solvent used is an alcohol.

4. Drugs characterized in that they contain at least one of the aminated derivatives of pyridazine of formula (I) or one of the corresponding pharmaceutically acceptable acids salts thereof.

5. Drugs according to claim 4, characterized in that they are active on the central nervous system.

6. Drugs according to any one of claims 4 or 5, characterized in that they are packed for oral administration and in that they contain from 0.01 to 0.50 g of derivative of formula I or a corresponding pharmaceutically acceptable acid salt.

**Claims for the Contracting State: AT**

1. A process for obtaining aminated derivatives of pyridazine of formula:

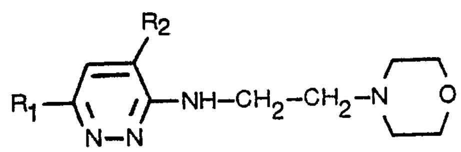

(I)

in which:

$R_1$ designates a 2-thienyl, 3-thienyl or cyclohexyl group;

$R_2$ represents an alkyl group having from 1 to 4 atoms of carbon, or an atom of hydrogen as well as the salts of said derivatives with the acids, characterized in that it comprises the steps of reacting a chlorinated derivative of formula:

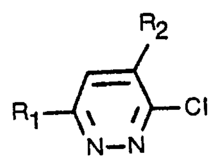

with an amine of formula:

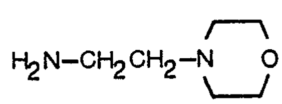

the reaction being carried out in a solvent, hot in the presence of a hydracid acceptor, and possibly of converting the derivative thus obtained into a salt thereof by action of an acid.

2. Process according to claim 1, characterized in that the solvent used is an alcohol.

3. Use of aminated derivatives of pyridazine of formula (I) and of the corresponding pharmaceutically acceptable acid salts thereof, for the preparation of drugs.

4. Use according to claim 3, for the preparation of drugs active on the central nervous system.

5. Use according to any one of claims 3 or 4, for the preparation of drugs, packed for oral administration and containing from 0.01 to 0.50 g of derivative of formula I or a corresponding pharmaceutically acceptable acid salt.

**Patentansprüche für die Vertragsstaaten: BE CH DE FR GB IT LI LU NL SE**

1. Aminierte Pyridazinderivate der Formel:

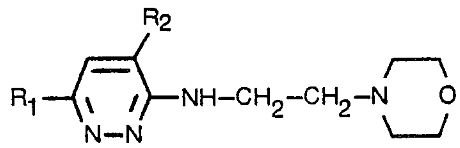

(I)

worin:
$R_1$ eine 2-Thienyl-, 3-Thienyl- oder Cyclohexylgruppe bezeichnet,
$R_2$ eine Alkylgruppe mit 1 bis 4 Kohlenstoffatomen oder ein Wasserstoffatom darstellt, sowie die Salze dieser Derivate mit Säuren.

2. Verfahren zur Herstellung der aminierten Pyridazinderivate nach Anspruch 1, dadurch gekennzeichnet, daß man ein chloriertes Derivat der Formel

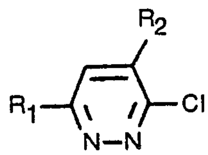

mit dem Amin der Formel:

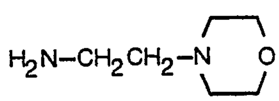

reagieren läßt, wobei die Reaktion in einem Lösungsmittel bei Hitze in Gegenwart eines Wasserstoffsäureakzeptors durchgeführt wird, und daß man gegebenenfalls die erhaltene Verbindung durch Einwirken einer Säure in eines ihrer Salze umwandelt.

3. Verfahren nach Anspruch 2, dadurch gekennzeichnet, daß das verwendete Lösungsmittel ein Alkohol ist.

4. Medikamente, dadurch gekennzeichnet, daß sie mindestens eines der aminierten Pyridazinderivate der Formel (I) oder eines ihrer entsprechenden pharmazeutisch akzeptablen Säuresalz enthalten.

5. Medikamente nach Anspruch 4, dadurch gekennzeichnet, daß sie aktiv auf das Zentralnervensystem sind.

6. Medikamente nach einem der Ansprüche 4 oder 5, dadurch gekennzeichnet, daß sie für eine Verabreichung auf oralem Weg konditioniert sind und 0,01 bis 0,50 g des Derivats der Formel I oder eines entsprechenden pharmazeutisch akzeptablen Säuresalzes enthalten.

**Patentansprüche für den Vertragsstaat: AT**

1. Verfahren zur Herstellung von aminierten Pyridazinderivaten der Formel

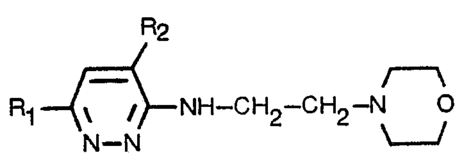

(I)

worin:
$R_1$ eine 2-Thienyl-, 3-Thienyl- oder Cyclohexylgruppe bezeichnet,
$R_2$ eine Alkylgruppe mit 1 bis 4 Kohlenstoffatomen oder ein Wasserstoffatom darstellt, sowie der Salze dieser Derivate mit Säuren, dadurch gekennzeichnet, daß man ein chloriertes Derivat der Formel

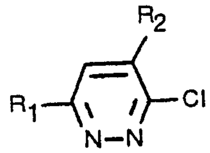

mit dem Amin der Formel:

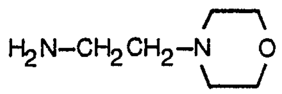

reagieren läßt, wobei die Reaktion in einem Lösungsmittel bei Hitze in Gegenwart eines Wasserstoffsäureakzeptors durchgeführt wird, und daß man gegebenenfalls die erhaltene Verbindung durch Einwirken einer Säure in eines ihrer Salze umwandelt.

2. Verfahren nach Anspruch 1, dadurch gekennzeichnet, daß das verwendete Lösungsmittel ein Alkohol ist.

3. Verwendung der aminierten Pyridazinderivate der Formel (I) und ihrer entsprechenden pharmazeutisch akzeptablen Säuresalze zur Herstellung von Medikamenten.

4. Verwendung nach Anspruch 3 zur Herstellung von Medikamenten, die aktiv auf das Zentralnervensystem sind.

5. Verwendung nach einem der Ansprüche 3 oder 4 zur Herstellung von Medikamenten, die für eine Verabreichung auf oralem Weg konditioniert sind und 0,01 bis 0,50 g des Derivats der Formel I oder eines entsprechenden pharmazeutisch akzeptablen Säuresalzes enthalten.